(19) Europäisches Patentamt / European Patent Office / Office européen des brevets

(11) **EP 2 426 509 B1**

(12) **EUROPEAN PATENT SPECIFICATION**

(45) Date of publication and mention
of the grant of the patent:
**22.03.2017  Bulletin 2017/12**

(51) Int Cl.:
***A61B 8/00*** *(2006.01)*       ***G01S 15/89*** *(2006.01)*

(21) Application number: **11009483.6**

(22) Date of filing: **21.06.2010**

(54) **Handheld ultrasound imaging apparatus and a method of producing an ultrasound image**

Handgeführte Ultraschallabbildungsvorrichtung und Verfahren zur Erstellung eines Ultraschallbilds

Appareil d'imagerie ultrasonore portable et procédé de production d'une image ultrasonore

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **23.06.2009  AU 2009902886**

(43) Date of publication of application:
**07.03.2012  Bulletin 2012/10**

(62) Document number(s) of the earlier application(s) in
accordance with Art. 76 EPC:
**10791047.3 / 2 421 441**

(73) Proprietor: **Signostics Limited**
**Thebarton, S.A. 5031 (AU)**

(72) Inventors:
• **El-Aklouk, Essa**
**Ferryden Park SA 5010 (AU)**
• **Bartlett, Stewart**
**Goodwood SA 5034 (AU)**
• **Parker, John**
**Roseville 2069 (AU)**

(74) Representative: **Beresford, Keith Denis Lewis**
**Beresford Crump LLP**
**16 High Holborn**
**London WC1V 6BX (GB)**

(56) References cited:
**US-A- 6 110 121        US-A- 6 123 670
US-A- 6 139 500        US-A1- 2002 050 169
US-A1- 2003 018 264    US-A1- 2006 250 046
US-B1- 6 547 731**

EP 2 426 509 B1

**Description**

**TECHNICAL FIELD**

The present invention relates to a real time medical ultrasound imaging system. In particular it relates to an embodiment of such a system employing a motor driven array.

**BACKGROUND ART**

Ultrasound is a non invasive technique for generating image scans of interior body organs. There are a number of types of real time ultrasound systems supporting a wide variety of ultrasound transducers. These systems can be divided into electronic systems such as phased array, curvilinear array, and linear array transducers which employ fully electronic techniques for beam forming and for directing the ultrasonic beam; and mechanical systems where a transducer or a transducer array is moved mechanically to direct the beam.

Mechanical scanners are traditionally the simplest and least expensive types of real time imaging systems. These systems utilize one or more piezoelectric crystals which transmit the sensing ultrasound signal, and receive the echoes returned from the body being imaged. To be effective, the ultrasound signal has significant directionality and may be described as an ultrasound beam. An electromagnetic motor is employed to move the crystal in a repetitive manner in order for the beam to cover an area to be imaged. The motor may be of any type, depending upon the movement characteristics required. Devices using stepping motors, DC motors and linear motors are known.

In general, mechanical ultrasound scanners employ one of two techniques for moving the beam and generating an image.

The first technique is the rotating wheel transducer where one or more crystals are rotated through 360° such that a beam emitted from the crystal would sweep out a circle. A sector of that circle constitutes the area to be imaged. The ultrasound signal is only transmitted and received while that sector is being swept out.

The second type of mechanical ultrasound scanner employs an oscillating transducer where a single crystal is moved back and forth by an electromagnetic motor such that the ultrasound beam emitted by the transducer sweeps out the region of interest.

Electromagnetic motors were invented more than a hundred years ago. These motors still dominate the industry, but must be considered to be at the limits of technological development. Performance improvements in mechanically driven ultrasound scanners cannot be expected from incremental improvements in such motors. Conventional electromagnetic motors are difficult to produce at the very small sizes which are desirable for use in a portable ultrasound probe. Electromagnetic motors are very hard to control with precision and have low position resolution. They are also characterized by high speed and low torque and have a slow response.

The limitations in electromagnetic motors cause a number of problems with the current single crystal mechanical transducers.

Such transducers are prone to registration artifacts which are spurious apparent reflections which occur because the motor is free running, and hence image frames produced from consecutive sweeps may not exactly align. This may also result in image jitter which can make the images fatiguing to view.

The motor is noisy and imparts a vibration to the ultrasound probe which is in contact with the patient. This may be disconcerting or uncomfortable for the patient.

In addition any form of Doppler imaging is not possible due to wobble and positional error in the motor.

Electronic systems which do not use motors, including phased array, curvilinear, and linear transducers overcome many of the problems with mechanical systems, including image registration, ability to perform Doppler imaging, vibration and noise. However, electronic systems have other shortcomings. They are more expensive to manufacture, have relatively high power consumption, and are relatively larger because, to achieve satisfactory performance, they include a large number of transducer elements and associated electronic channels.

A system with a transducer array and a motor is known from US 2002/0050169.

**DISCLOSURE OF THE INVENTION**

According to this invention there is proposed a handheld ultrasound imaging apparatus according to claim 1 and a method of producing an ultrasound image according to claim 6. Preferred embodiments are set ouf in the dependent claims.

**BRIEF DESCRIPTION OF THE DRAWINGS**

Figure 1 a shows a transducer of the prior art.

Figure 1b shows a diagrammatic representation of an ultrasound field of a transducer of the prior art of the type illustrated in Figure 1 a.

Figure 2a shows a diagrammatic representation of a linear segmental array transducer of the prior art.

Figure 2b shows a diagrammatic representation of an ultrasound field of a transducer of the prior art of the type illustrated in Figure 2a.

Figure 3 shows an ultrasound scanning apparatus incorporating the invention.

Figure 4 shows a transducer slider of the scan head of Figure 3.

Figure 5a shows a diagrammatic representation of an ultrasound transducer array according to the present invention.

Figure 5b shows a diagrammatic representation of the motion of the ultrasound transducer slider of Figure 4.

Figure 6a shows a diagrammatic representation of a curvilinear transducer array of the invention.

Figure 6b shows a diagrammatic representation of the motion of the ultrasound transducer slider of Figure 6a.

Figure 7a,b,c show diagrammatic representations of three possible arrangement of a transducer array slider in accordance with the invention.

Figure 8a shows a cross section view of a transducer head incorporating the invention using a linear ultrasonic motor.

Figure 8b shows a cut away view of an ultrasound probe unit scan head incorporating a rotary motor.

Figure 8c shows a diagrammatic representation of an embodiment incorporating a rotary ultrasonic motor driving a ring transducer array directly.

Figure 9 shows a system block diagram of an ultrasound probe unit scan head having a curvilinear shaped transducer and including a USB interface connection to a host display.

Figure 10 shows a system block diagram of an ultrasound probe unit scan head having a single channel ultrasound element and including a USB interface connection to a host display.

Figure 11 shows a system block diagram of an ultrasound probe unit scan head having a curvilinear shaped transducer and including a gyroscope and a USB interface connection to a host display.

Figure 12 shows a system block diagram of an alternative embodiment of the ultrasound probe unit scan head including a wireless interface to a host display.

Figure 13 shows a system block diagram of an ultrasound probe unit scan head having an integrated display.

Figure 14 is a block diagram of an FPGA timing and control unit.

Figure 15 is a block diagram of the DSP software of the system of Figure 1.

Figure 16a, 16b, and 16c illustrate the scan conversion requirements for different transducer shapes which may be incorporated in apparatus of the present invention.

Figure 17 illustrates a Doppler processing window.

## BEST MODE FOR CARRYING OUT THE INVENTION

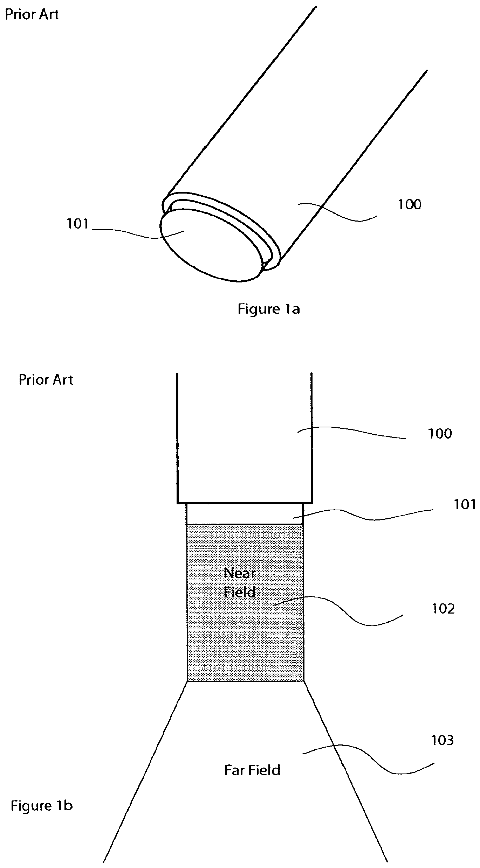

Figure 1 a shows a diagrammatic representation of a simple, single crystal ultrasound transducer as used in early ultrasound scanners. This has a probe unit body 100, which supports a circular ultrasound crystal 101. This crystal is relatively large.

Figure 1b shows the unit of Figure 1 a in cross-section, with a representation of the ultrasound field produced by the transducer. This arrangement has several desirable features. The ultrasound beam produced by the crystal has a relatively long, even near field 102, before the beam diverges in the far field 103. The lateral resolution is best in the non-divergent field, so the long near field is advantageous. The far field also diverges relatively slowly, improving the lateral resolution in this area when compared to transducers with a more rapidly diverging far field.

However, such an arrangement produces only one scanline. In order to scan a region it is necessary to move the transducer over an area to be scanned, obtaining scanlines at different positions, and to keep very accurate track of the movement so that the scanlines can be displayed in correct spatial relation to form an ultrasound image. This was achieved using large articulated arms, with exact position control.

These were effective, but severely restricted the ways and places in which ultrasound could be deployed.

In order to avoid the need for such arms, it would be possible to use an array of crystals to form a transducer. This would allow for an area to be scanned without the cumbersome articulated arm, or the need to keep track of the position probe, since the positional relationship between the crystals, and hence the scanlines, would be fixed. However, the large crystals mean that relatively few scanlines would be gathered from a scan region, significantly reducing the utility of the image.

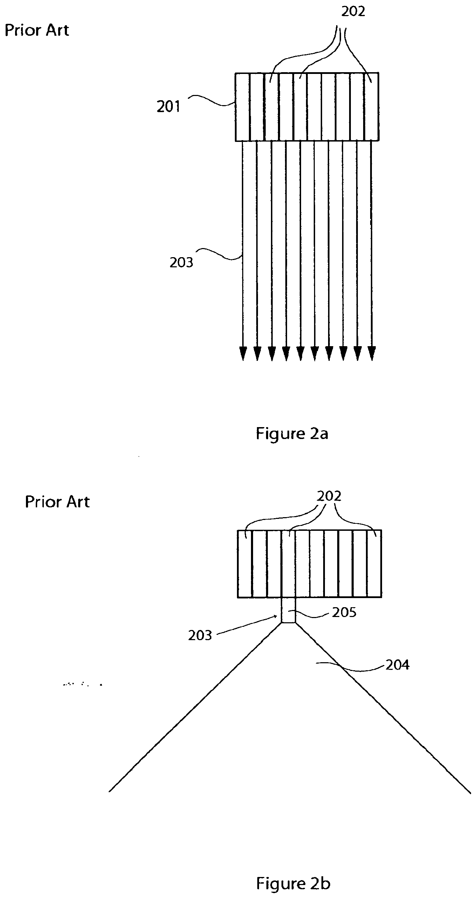

In practice, the problem was solved by using a segmental linear array, as shown diagrammatically in Figure 2. The segmental linear array consists of a transducer made from a single, rectangular piece of piezoelectric material 201. This piezoelectric material is divided into a number of individual transducer elements 202. Each element is separately electrically connected to the ultrasound driving electronics, and may be individually excited to produce a scanline 203.

Each individual element is relatively narrow, compared to the width of the crystal of Figure 1, allowing more scanlines to be collected from a given region.

However, the smaller transducer element has a less desirable beam shape. As shown in Figure 2b, the beam 203 has a shorter near field 205, and a more divergent far field 204. This leads to reduced lateral resolution, which is undesirable.

The prior art includes solutions involving firing the elements in groups to improve beam shape, and firing the elements in very specific patterns to form and electronically steer a beam across the area to be scanned. In prior art the signal of each transducer element is combined with a number of adjacent elements, usually 32, to produce one scanline.

All of these solutions lead to compromises of the near-ideal beam shape provided by the circular crystal transducer of the original devices. Sophisticated control and signal processing techniques are used to counter these problems, but these introduce further cost and complexity. These solutions also introduce very great complexity and cost into the physical design of the transducers, with the need to provide large numbers of transducer elements each requiring precise, matched electrical connections.

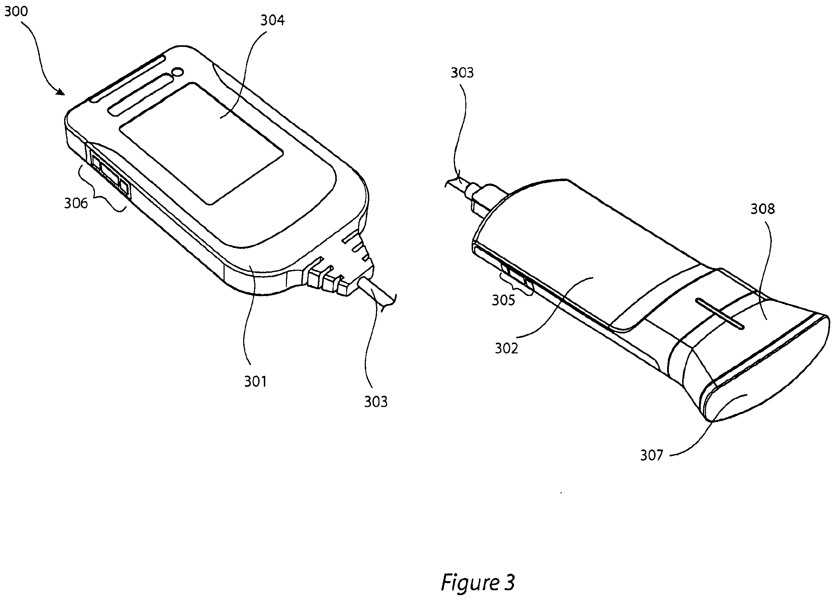

Now referring to Figure 3, there is shown a view of an ultrasound imaging device 300 incorporating an embodiment of the invention. This includes a display unit 301, and a probe unit 302. These are connected by communications cable 303. The display unit has a display 304. The display screen may be a touch screen allowing a user to control the functionality of the display unit and the probe unit. In the illustrated embodiment, control members 306 are provided on the display unit, in the form of push buttons and a scrollwheel. Control members 305 are provided on the probe unit. Either of both of these control members may be absent.

The probe unit 302 includes or is connected to a probe unit scan head 308. The scan head 308 includes a transducer which may be, without limitation, an array transducer having multiple transducer elements, a single element transducer or an array of separate, individual transducers.

The scan head 308 includes a front panel 307 which contacts the patient during scanning. This front panel is acoustically transparent. It is desirable to provide the best possible acoustic coupling between the transducer elements and the body to be imaged.

In use, the probe unit is held against the body of a patient adjacent to the internal part of the body which is to be imaged, with the front panel 307 in contact with the patient's skin. Electronics in the probe unit stimulate the emission of ultrasound energy from the transducer. This beam is reflected back to the transducer as echoes by the features to be imaged. The transducer receives these echoes which are amplified and converted to digital scanline data.

The device 300 is for handheld use. The probe unit and the display unit are of a size and weight which may be comfortably held in a user's hand. The probe unit weighs less than 500 grams, and preferably less than 200 grams. In a preferred embodiment, the device in total weighs less than 500 grams. The probe unit and the display unit may be shaped in a variety of ways, but for ease of handheld use, no linear dimension of either unit exceeds 15 centimetres. Preferably, no linear dimension exceeds 12 centimetres. In a further preferred embodiment, no linear dimension exceeds 10 centimetres.

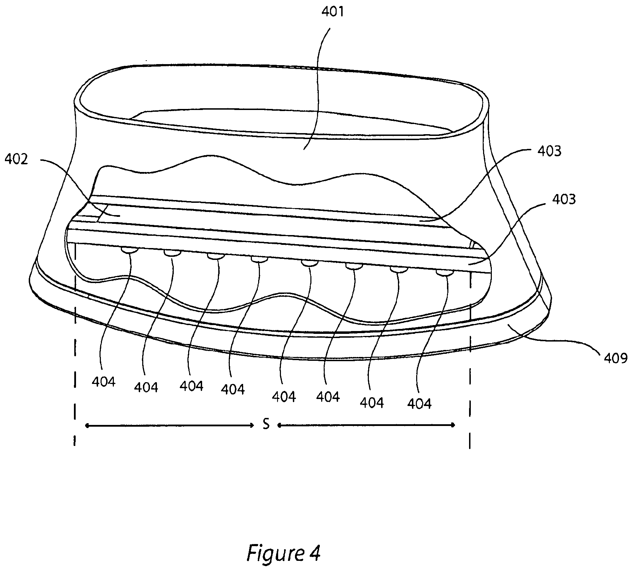

Figure 4 shows a scan head of the ultrasound scanning device probe unit of Figure 3. There is a scan head 401 which includes a transducer slider 402, having transducer crystals 404. There may be as few as one transducer crystals, with the maximum number being limited only by practical considerations of cost, size and complexity. The slider is guided and constrained to move in a reciprocating fashion by guide rails 403.

In use the slider is driven in a reciprocating fashion such that the beams from the transducers cover an area to be imaged. The slider may be driven in said reciprocating motion by any convenient means.

In a preferred embodiment motor moves the transducer such that the ultrasound beam or beams sweep out an area to be imaged. Electronics for control of the motor are provided in the probe unit. The motor may be a linear or a rotary motor.

In a preferred embodiment, the linear motor is a linear ultrasonic motor.

In a further embodiment, the rotary motor is an ultrasonic motor.

In a preferred embodiment, the linear motor is a linear ultrasonic motor. The linear ultrasonic motor can either be of a standing wave type or a propagating wave type, as will be known to those skilled in the art of ultrasonic motors.

Ultrasonic motors work by stimulating vibration in a vibratory portion. This may be either the stator or the slider, but is most usually the stator. The slider or forcer is the equivalent of the rotor in a rotary motor. In an electromechanical motor contact between the slider and the stator is undesirable, but in an ultrasonic motor such contact is necessary to provide the motive force. The stator and the slider are held together, usually by spring loading.

There are two main types of ultrasonic motor, the standing wave type and the propagating wave type.

In the standing wave type, a standing wave is induced into the stator. There will be points on the surface of the stator with the same desired vibration direction and amplitude. At these points, contact tips or pushers are applied or formed. Frictional contact with the slider occurs only at these points, which results in the slider being given a chain of angled micro-pulses, pushing the slider in the desired direction.

By comparison, the propagating-wave type combines two standing waves with a 90 degree phase difference both in time and in space. A travelling wave is induced in the stator. In this case any surface particle of the stator travels in an elliptical locus due to the coupling of longitudinal and transverse waves. Contact between the stator and the slider is along the length of the slider, which is driven continuously.

The term "ultrasonic motor" is used throughout this specification. Other terms may be used for devices having the same principle of operation but varying in size, configuration and/or application. These terms include, without limitation, piezomotor, piezoelectric actuator, piezoactuator, and ultrasonic actuator. The term "ultrasonic motor" as used in this specification covers all of these and any other possible terminology which may be used to describe ultrasonically driven moving devices which may be used to perform the invention.

In an embodiment, the motor is an electromagnetic motor.

An acoustically matched lubricant may be provided on the interior side of the front panel 409 of the scan head filling the gap between the transducer elements 404 and the panel 409.

In a further embodiment a strip of solid material having a low coefficient of friction and acoustic matching properties is provided on the interior side of the front panel 409 of the scan head filling the gap between the transducer elements 404 and the panel 409.

In this embodiment, the transducer array is an array of eight piezo-electric elements. In other embodiments, other numbers and other types of transducer elements may be employed. In particular embodiments, capacitive micro-machined ultrasonic transducers (CMUTS) may be used.

In a conventional linear array transducer ultrasound probe, each transducer element is fired in a group of a number of adjacent elements, usually 32, to produce one scanline. The lateral distance between scanlines is proportional to the linear distance between successive transducer elements in the array.

To provide a higher resolution image, without sacrificing image coverage; or to provide a wider image coverage without sacrificing resolution, requires more scanlines and hence more transducer elements. Providing additional elements is expensive. The larger transducer is itself more expensive, but the additional wiring and electronics required to access and control the additional elements (usually called channels) is also expensive.

An array transducer with few elements, set relatively far apart gives good image width for less cost, but sacrifices the number of scanlines in the image and hence sacrifices lateral resolution.

Conventional linear array systems have transducers with a large number of crystals, each cut in a rectangular shape. This allows for the closest possible spacing of the transducer array elements, giving close spacing of scanlines without moving the array. However, this generates an acoustic beamshape with a non-symmetrical pattern.

It is desirable to use circular crystals, where the beamshape is circular and symmetrical. It is also desirable for the crystals to be relatively large, to give the best depth of field. For conventional ultrasound systems, these requirements are conflicting.

Using an array transducer with few elements, set relatively far apart and moving the transducer, allows the relatively wide gap between the transducer elements to be "filled in" with scanlines, thereby providing similar performance and image size to systems with more channels, at greatly reduced cost and complexity. The scanline density is not restricted by the physical size of the transducer elements or the spacing or number of elements. Larger crystals with desirable geometry may be used.

The transducers of the invention, form a sparse array, defined as an array of transducers wherein the distance between adjacent transducers or transducer elements is greater than the minimum separation of adjacent scanlines required to produce an ultrasound image of a desired resolution.

Using a sparse array of individual transducers makes the use of circular transducers in a linear array practical.

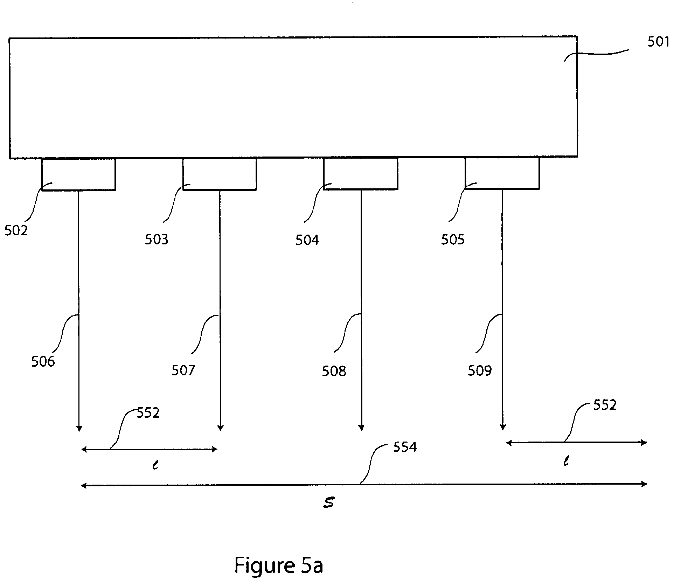

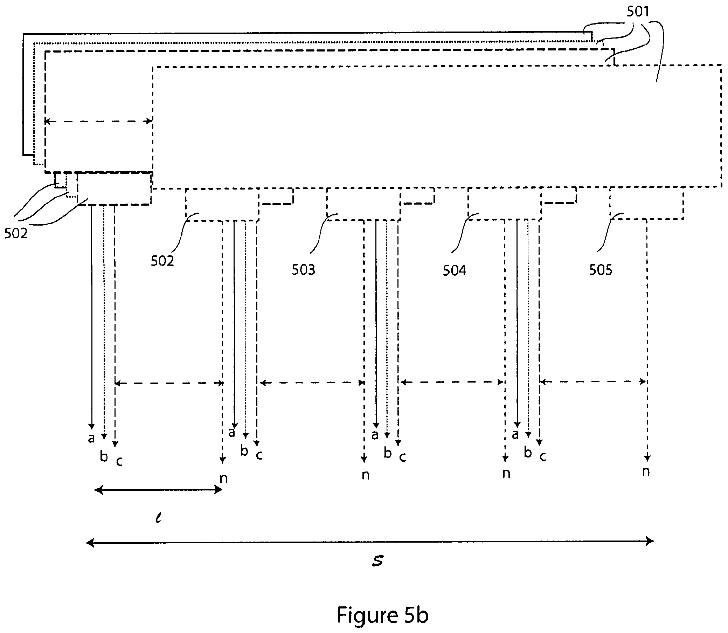

Figure 5 shows a diagrammatic representation of an ultrasound transducer array of the current invention and a manner of use. Referring to Figure 5a there is a transducer slider 501 which supports or is integrally formed with, ultrasound transducers 502-505. Each transducer is able to transmit ultrasound energy and to receive the resultant echoes from a body to be imaged to gather one scanline 506-509. There may be as few as one transducer crystals, with the maximum number being limited only by practical considerations of cost, size and complexity. The transducers form a sparse array.

The transducers are arrayed at a separation 1 552. The transducer slide may be moved such that the width of coverage of the linear array is the distance designated as S 554. This is the total width able to be covered by the array over the full range of movement.

The slider is moved, continuously or in steps, the distance I. The transducer elements transmit and receive ultrasound energy whilst moving or at each step in order to receive a scanline which is a series of echo intensity values returned from features at various depths along a line running into the body to be imaged. The slider is driven in a reciprocating fashion such that the beams from the transducers cover an area to be imaged. The slider may be driven in said reciprocating motion by any convenient means.

Figure 5b shows the effect of moving the transducer slider. The series of dotted line diagrams of the transducer slider 501 represent the movement of the ultrasound slider through the distance I. Each of the transducers is driven to transmit and receive a series of scanlines *a,b,c ...,n*. The combination of these *n* scanlines for all of the transducers produces a scanset which covers the distance S. The distance S is the width of the B mode ultrasound image which may be produced by the probe unit.

All of the scanlines captured during a single traverse of the transducer of the distance l form a scanset. The scanlines in each scanset are processed and displayed on display screen 304 as an ultrasound image.

The image displayed as an ultrasound scan image is essentially a display of a series of scanlines in the same spatial relationship as they were acquired by the transducer. In order to be displayed as part of a scan image, each scanline must include, or be associated with, data indicating the relative location of the transducer element which received the scanline data at the time it was received. This can be done by any means known in the art. A method based on knowledge of the rotational or linear position of the motor may be used, or the position of the slider may be directly monitored by a device such as a linear encoder.

Each scanset corresponds to a single frame ultrasound image. The distance moved by the transducer slider between successive firings of the same transducer, for example between the acquisition of scanlines a and b and between the acquisition of scanlines *b* and *c* is sufficiently small to provide the desired image resolution.

Post-processing may be applied to enhance the image in a variety of ways.

The slider is moved in a reciprocating fashion. A new scanset is obtained on each pass over the area to be imaged. Scansets are displayed sequentially as they are received to form a real time display.

In a prior art linear array transducer ultrasound probe, as shown in Figure 2, the lateral distance between scanlines is proportional to the linear distance between successive transducer elements in the array.

To provide a higher resolution image, without sacrificing image coverage; or to provide a wider image coverage without sacrificing resolution, requires more scanlines and hence more transducer elements. Providing additional elements is expensive. The larger transducer is itself more expensive, but the additional wiring and electronics required to access and control the additional elements (usually called channels) is also expensive.

An array with few elements, set relatively far apart gives good image width for less cost, but sacrifices the number of scanlines in the image. Moving the transducer as described allows the relatively wide gap between the transducer elements to be "filled in" with scanlines, thereby providing similar performance and image size to systems with more channels, at greatly reduced cost and complexity. The scanline density is not restricted by the physical size of the transducer elements or the spacing or number of elements.

In order to be displayed as part of a scan image, in the spatial relationship in which the scanlines were received, each scanline must include, or be associated with, data indicating the relative location of the transducer element which received the scanline data at the time it was received. This can be done by any means known in the art. A method based on knowledge of the rotational or linear position of the motor may be used, or the position of the slider may be directly monitored by a device such as a linear encoder. This data may be transmitted to the display unit as part of the scanline data; as a separate data stream; or the information may be inferred and calculated by a processor in the display unit.

The image captured by the linear sparse array of Figure 5 has a maximum width of S 554, being the width of the transducer slider plus the distance travelled by the slider. This can be increased by increasing the width of the slider 501, but this increases cost. Further, a larger slider requires a larger probe unit, which is undesirable form an ease of use perspective.

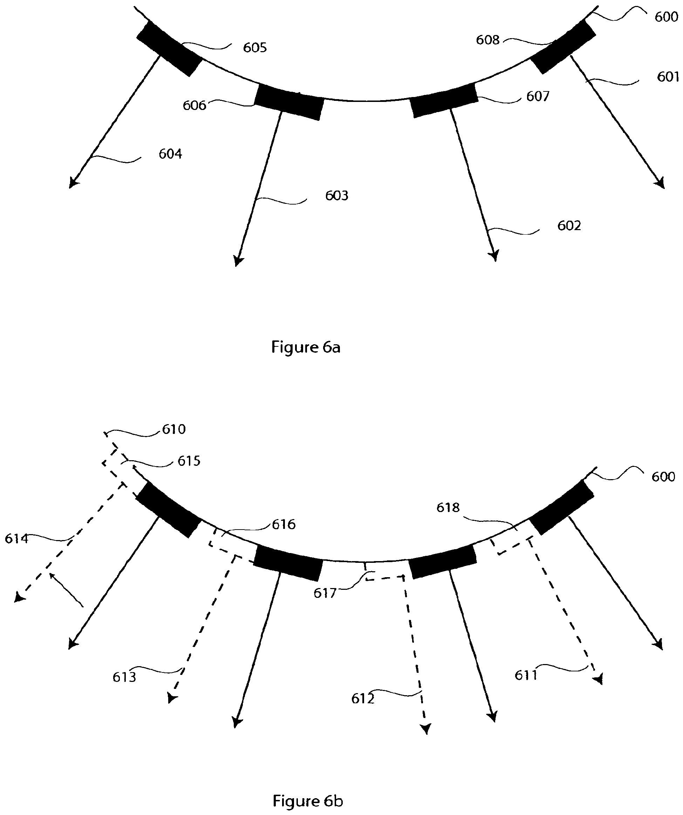

Figure 6 shows a diagrammatic representation of the invention applied to a curvilinear scan head form factor.

There is a transducer slider 600 to which are attached one or more transducers. The illustrated embodiment has four transducers 605-608. The transducer slider is shaped as the arc of a circle. Each transducer is able to transmit and receive ultrasound energy in order to produce a single ultrasound scanline 601-604. The transducer slider is incorporated in and forms part of an ultrasound probe unit as illustrated in Figure 3.

In use, the transducer slider is rotated about an axis through the centre of the circle of which the transducer forms an arc. This may be done by use of a pivot arm with a pivot point at said centre. Alternatively the transducer slider may be guided by arc-shaped guides. Other means to constrain the slider to move along the path of the arc of the circle may be employed. The power to rotate the slider is provided by a motor, which in a preferred embodiment is an ultrasonic motor.

Figure 6b shows the rotated transducer slider 610 with transducers 615-618 producing scanlines 611-614, superimposed on the original position of the slider 600.

The placement of the transducers on the outside of a circle means that the overall area covered by the ultrasound beams is a sector of a circle. This allows broader coverage than the width of the transducer probe unit. The greater the curvature of the slider the greater is the amount by which the width of the scanned area can exceed the width of the probe unit.

The received scanlines are processed for display as an ultrasound image having a form factor similar to the well known sector of a circle ultrasound image. The transducer slider is reciprocated by the motor to achieve real time coverage of the area being imaged. Substantially all of the scanlines captured during one reciprocation are displayed to form an image frame. Successive display of the frames as the scanlines are received gives a real time display. Real time frame rates on the order of 20 frames per second are achieved. Higher frame rates are possible.

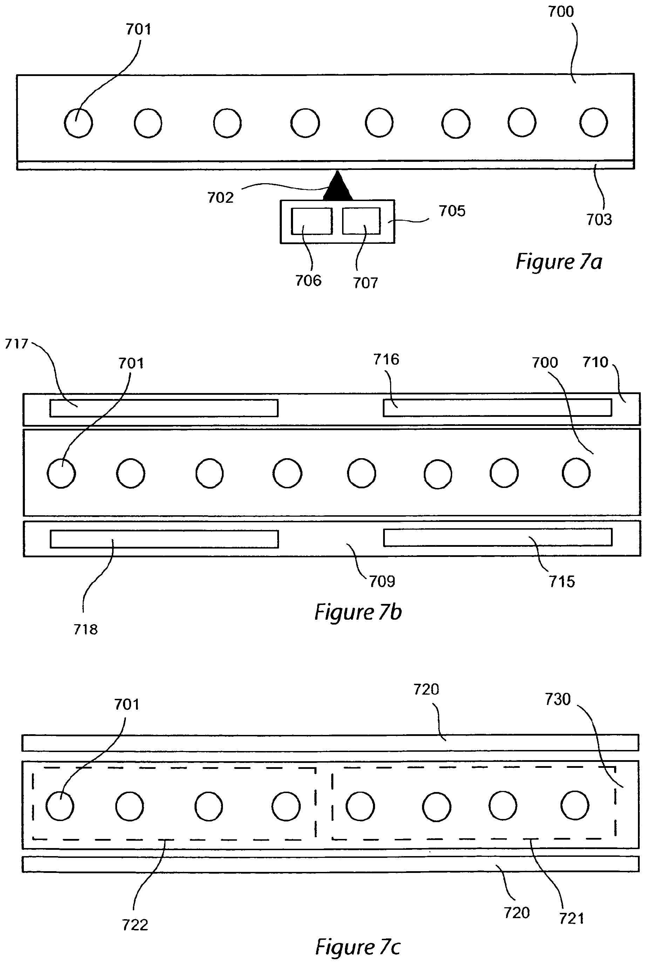

Figure 7 shows three possible alternative embodiments for the transducer array slider for embodiments of the invention wherein the transducer slider is the slider of an ultrasonic motor. There is a transducer array slider 700, with individual transducers 701 mechanically attached to, or integrally formed with, the slider.

As shown in Figure 7a, the slider may include a friction strip 703 integrally formed or mechanically attached to the body of the slider 700. This friction strip is in contact with contact tip or pusher 702. This contact tip is driven by piezoelectric driver 705. The piezoelectric driver is excited by an appropriate signal such that vibration of the driver against the friction strip drives the slider in a reciprocating fashion. The piezoelectric driver includes two electrodes 706, 707 attached to a piezoelectric material. There is also a third, common electrode, (not shown) on the bottom of the piezoelectric material. Applying a driving signal to one of the electrodes, while the other is left floating, causes a standing wave to be set up in the driver which causes the contact tip to vibrate such that the slider is driven in one direction. Similar excitation of the other electrode causes the slider to be driven in the reverse direction. Springs or other means (not shown) force the friction strip against the contact tip. The slider is constrained by a frame or guides (not shown) to move in a reciprocating fashion.

In an alternative embodiment, as shown in Figure 7b, the slider 700 may be constrained and guided by guide rails 709, 710 which bear against the edges of the slider 700, forming part of an ultrasonic motor. There are piezoactuators 715-718 rigidly attached to the guide rails. The piezoactuators have an electrode and piezoelectric material. Application of an electrical signal at ultrasonic frequency causes the piezoelectric material to vibrate, and to induce vibration in the attached guide rail.

The slider edges and the rails 709,710 in pairs form the contact surfaces of a linear ultrasonic motor. Vibration is induced in the rails by electric signals applied to electrodes 715-718. A travelling wave is induced into the guide rails and the slider moves by "surfing" on this wave. The phase of the waves induced determines the direction of the travelling wave and hence the direction of travel of the slider.

In all embodiments, the rails also provide the physical support and linear guidance for the slider. There are also provided springs or similar means (not shown) to hold the contact surfaces in contact.

A further embodiment is shown in Figure 7c. The ultrasonic motor slider 730 is constructed of a vibratory material, in this embodiment a piezoelectric material. Other ultrasonic vibratory structures could be employed. The slider is supported and guided by guide rails 720. Attached to the slider are excitation electrodes 721, 722. A common drain electrode (not shown) is also provided. A voltage signal at an ultrasonic frequency is applied to the electrode 721 to induce into the slider an ultrasonic wave to drive relative motion in one direction between the slider 700 and the rails 720. A voltage signal applied to electrode 722 is used to drive the slider in the opposite direction.

As illustrated, the slider is the slider of an ultrasonic motor, but it should be understood that the transducers may be supported by a backing element, which is mechanically attached to the slider of an ultrasonic motor. In this case, the size of the motor slider and that of the backing element have no essential relationship.

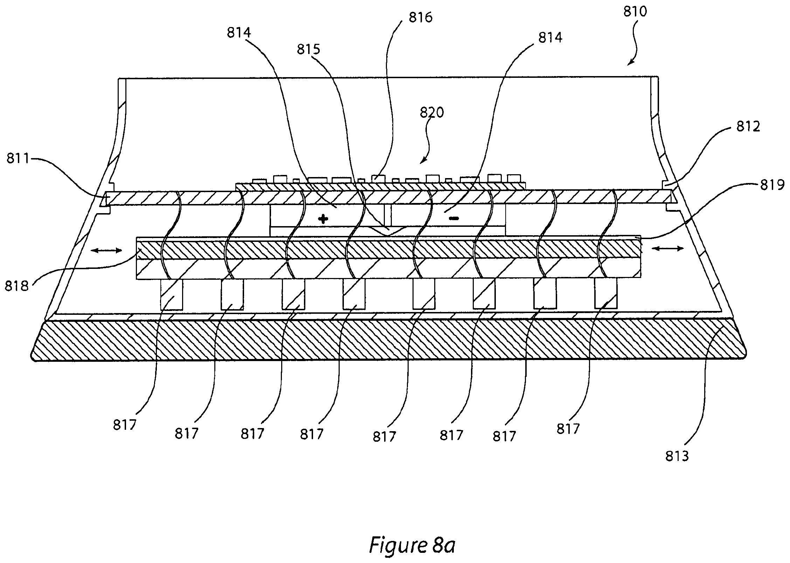

Figure 8a shows an embodiment of the invention wherein the slider of the ultrasonic motor is separate from the transducer array. There is a scan head 810 which includes an ultrasonic motor 820, consisting of excitation electrodes 814, vibratory driver 815 and slider 818 held by a frame and guide rails. The slider includes a friction layer 819 which facilitates drive friction between the vibratory driver 815 and the slider 818. The motor 820 is attached to a circuit board (PCB) 811, which is positioned and supported within the scan head 810 by locating guides 812. The PCB includes or has attached to it, ultrasonic motor control circuitry 816. This control circuitry supplies signals to the excitation electrodes 814 in order to drive the motor 820.

Attached to the slider is transducer array 817. The transducers making up the array may be of any suitable type. The transducers are attached to a backing element, which is mechanically connected to the slider 818 of the motor. This arrangement simplifies the assembly and manufacturer of the system, as the main PCB is mechanically connected to the motor and ultrasound transducers, and the motor can operate independently of the enclosure.

The transducers are electrically connected to circuitry on the PCB 811 which excites the transducers to produce ultrasonic output and receives and processes the electrical signals returned from the transducers.

In the illustrated embodiment, a non-liquid friction reduction means is provided to allow the transducer array to slide easily over the acoustic window 813 which, in use, is in contact with the patient. This may be, without limitation, a solid lubricant, or the material of the acoustic window may inherently provide low friction.

An acoustically matched lubricant may be provided on the interior side of the front panel of the scan head filling the gap between the transducers and the panel.

In a further embodiment a strip of solid material having a low coefficient of friction and acoustic matching properties is provided on the interior side of the front panel of the scan head filling the gap between the transducers and the front panel.

In other embodiments, a flexible, liquid-proof, membrane may be provided between the transducer array 817 and the motor 820, forming a closed compartment with the acoustic window. This enclosed compartment encompasses the transducer array. With the motor thus protected from liquid, the transducer array may be immersed in a liquid, which provide acoustic coupling between the transducers of the transducer array and the acoustic window. In this case the transducer array need not touch the acoustic window in use. Any gap between the transducers and the acoustic window will not cause significant reverberation in the received ultrasound signal, since the gap will be filled with the acoustic matching liquid.

In the illustrated embodiment, the transducer array is an array of eight piezo-electric transducers. In other embodiments, other numbers and other types of transducer elements may be employed. In particular embodiments, capacitive micro-machined ultrasonic transducers (CMUTS) or other MEMS based transducers may be used.

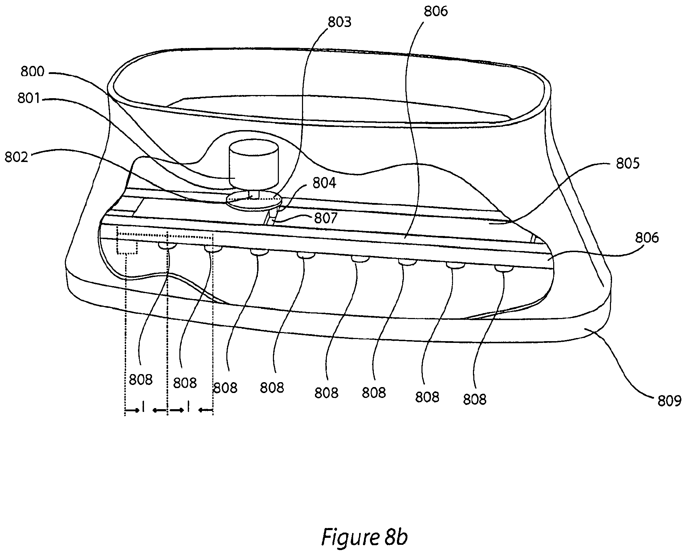

Referring to Figure 8b, the slider may be driven by a rotary motor, which in a preferred embodiment is an ultrasonic motor.

The range of the reciprocating movement, being the linear movement of the slider 805, is approximately equal to d, the diameter of the drive disk 803. The number of transducer elements 808 is N. The linear distance between successive transducer elements is I.

In order to achieve full coverage of the area to be scanned with the same density of scanlines, it is necessary that I should be less than or equal to d. Preferably I should be approximately equal to d.

Preferably:

$$d \sim \; l \; = S/N$$

The number of transducer elements used can vary depending on a number of factors including coverage area, motor speed, disk size and motor response time. In the illustrated embodiment a 7.5 MHz scan head with eight 3 mm circular crystals 808 mounted on the slider 805 combined with a 6 mm width disk 803 provides a scan head with 48mm (8x6 mm) coverage.

The rotational motion of the ultrasonic motor 800 is translated into reciprocating motion. There is a drive disk 803 which has an off centre pin 804 protruding from one face. The disk is attached to a shaft 802 driven by the rotary motor. The slider 805 to which the transducer element array is mounted includes a slot 807. The off centre pin 804 of the disk is located into the slot. Rotation of the disk causes the pin to impart a reciprocating motion to the slider. The slider is restrained from motion other than reciprocation in two dimensions by rails 806 on each side.

The part of the scan head containing the transducer array, may be filled with a liquid medium to eliminate any air interfaces between the moving transducer elements 808 and the protective front panel 809 of the scan head. The transducer array compartment is separated from the motor 800 by a seal 801 around the motor rotor 802. This prevents the liquid medium from entering and potentially damaging the motor.

In further embodiments the transducer may be implemented such that the array is moved in a curvilinear pattern. This is illustrated diagrammatically in Figure 6.

In such embodiments, the slider is shaped as a circle segment, while the guide rails, if used, and the front panel are shaped as arcs of a circle. Either a rotary or linear motor may be used to drive the slider. For a linear motor, the slider and guides may form a linear motor, or a separate linear motor may be provided. Where a rotary motor is used, the slider may have a slot which accepts the offset pin drive as for the illustrated embodiment using a rotary motor. The slot will be trapezoidal in cross section to accommodate the fixed orientation of the drive pin. Where the radius of curvature of the slider segment is sufficiently small, a rotary motor may move the slider by directly driving a rotor arm.

A rotary motor shaft may be used to drive a fully annular slider. This slider will have transducers on the outer face. These transducers may be grouped to form a curvilinear array in the form of an arc of a circle, or they may be evenly distributed around the circumference of the annular slider.

In a preferred embodiment, the motor moves the transducer array slider across the width of the area to be scanned in a reciprocating fashion. Each transducer element is fired in turn. Each firing results in a single scanline of data being collected. The delay between firing each transducer element should be at least sufficient to allow for the return of echo from that depth within the body being imaged where it is desired for an image to be obtained. The firing process repeats. Generally, each transducer element will be fired many times in the time it takes for a single reciprocation of the slider. Preferably the firing is timed such that on each successive reciprocation, the firing of each transducer element is in substantially the same places with respect to the probe unit body.

Preferably, the delay between successive firings of a transducer element is related to the width of the beam from the transducer elements such that the entire length of the area being scanned is covered by a focused beam.

In an alternative embodiment, the system operates by firing each transducer element individually with the transducer slider at rest to acquire a set of scanlines. The motor then moves the slider a step, with the slider coming to a halt before the transducer elements are again fired acquiring a second set of scanlines. This continues until the distance 1 is covered. The process repeats.

The size of the step is determined to provide the required scanline density over the area to be imaged. It may be varied in use in order to optimise characteristics of the scan system such as fame rate or motor speed.

All of the acquired scanlines for a single full traverse of the slider are then combined to form one frame of a real-time ultrasound display. In order to be displayed as part of a scan image, each scanline must include, or be associated with, data indicating the relative location of the transducer element which received the scanline data at the time it was received. This can be done by any means known in the art, for example, the position of the slider may be directly monitored by a device such as a linear encoder.

The number of steps used is determined by the scanline density required and the magnitude of the transducer element separation l.

The number of transducer elements used can vary depending on a number of factors including coverage area, motor speed, scanline density desired and motor response time.

All of the scanlines acquired in one full transverse movement of the transducer constitute a single frame for display. This means that the firing rate of the transducer as a whole is determined as the product of the number of elements, the frame rate and the number of scanlines required to cover the distance between the elements at the desired density.

Other element firing regimes are possible. Where this can be done without undue interference between the scanlines, more than one element may be fired at a time. Without limitation, a means of achieving this is for the transmit pulses to be coded, for example with Barker, Golay, or Gold codes, such that the correlation between pulses from separate transducers is extremely low.

In a further embodiment, the slider length is less than the image width S, by an amount greater than the separation of the transducer elements l. It may be much less. The scan head and rails cover the full image width. The slider is driven in incremental steps to cover the required image width. In this embodiment, the elements of the transducer array may be arranged at such a separation that adjacent elements give the desired scanline density. In this case, the slider may move an amount equal to its full length between each full firing of the array.

In the most general case, the transducer may consist of a single element. This is not the currently preferred embodiment since one of the limitations of ultrasonic motors is that they have low linear and rotational speeds. This poses a constraint on the speed the ultrasonic crystal can scan the region of interest and hence limits the systems frame rate. Thus using an array incorporating more than one crystal which decreases the commuting distance of the crystals and reduces the motor speed requirements is currently preferred, but further improvements in motor design may make this alternative practical.

Pulsed Wave Doppler imaging is achieved by electronically stimulating a transducer element to produce a pulse of ultrasound and then to listen for echoes before another pulse is generated. This is done rapidly at a single location, and the variable frequency shift in the returned echoes is used to detect movement in the feature being imaged, for example blood flow. These modes are not possible with motor driven transducer systems of the prior art, since they cannot be held stationary nor rapidly and accurately repositioned.

This can be achieved by the apparatus of the current invention because ultrasonic motors have excellent response time and can be positioned extremely accurately, with errors in the nanometre range. This allows the transducer to be completely stationary at a known position when sending and receiving ultrasound pulses.

Color Doppler combines real time imaging with Doppler imaging. Real time images are displayed for the full image area at the same time as Doppler information is displayed for one or more scanlines. This is not possible for any prior art system with a moving transducer, since Doppler requires that the transmitting element be stationary in a known position and real time scanning requires that the transducer be moved.

In an embodiment providing color Doppler functionality, the transducer array is controlled to move to produce a real time moving image on a display for a user. The user is able to select an area of the displayed image for acquisition of color Doppler information. A selected element or elements of the transducer, while located above the selected area, fires the required number of pulses and receives the necessary echoes to acquire the movement information, being the color Doppler frequency shift data. The transducer array is stationary whilst the Doppler information is acquired. The real time scan operation then continues, with the transducer being moved by the ultrasonic motor. Real time image scanlines are acquired whilst the transducer array is moving. When transducer has moved a selected distance, the transducer stops and the selected element again fires multiple pulses, acquiring Doppler information. This continues until a full real time image frame has been acquired, along with Doppler information for a subset of that frame. The process then repeats to acquire a real time image display.

The color Doppler and real time information are combined into a single display, as is known in the art.

The fast response and excellent positioning accuracy of ultrasonic motors allows the transducer to come to complete stop, fire the required number of ultrasound pulses, listen to the echoes, and then move to the next line while simultaneously performing real time imaging. Generally the real time frame rate will be reduced while color Doppler is in use.

Embodiments using ultrasonic motors may also use a curvilinear array where the linear slider is replaced with a curvilinear slider with N transducer elements mounted on it or machined as part of the slider. The rails and the front panel would also be manufactured in a curvilinear construction. Ultrasonic motors are readily manufactured in different shapes and sizes. In embodiments where the slider itself is part of the motor, no special arrangement or shaping of the slider is necessary to allow it to be driven by an external motor.

In alternative embodiments, a linear ultrasonic motor separate from the curvilinear slider may be used to move a curvilinear slider through an arc of a circle.

The slider may be driven through a full circle in a continuous revolution. In this case, the transducer would be active only when it was over the body to be scanned. This is especially relevant for micro curvilinear shaped scan heads, which have a relatively tight radius of curvature so as to enable imaging between ribs. Two or more transducers may be provided, each having a different operating frequency, allowing simultaneous or separate scanning at different frequencies. Doppler imaging would be possible as described for other embodiments.

In an embodiment, it is possible for the rails to be formed into a circle, and the slider driven in a continuous revolution. In this case, the transducer would be active only when it was over the body to be scanned. This is especially relevant for micro curvilinear shaped scan heads, which have a relatively tight radius of curvature so as to enable imaging between ribs. Two or more transducers may be provided, each having a different operating frequency, allowing simultaneous or separate scanning at different frequencies. Doppler imaging would be possible as described for other embodiments.

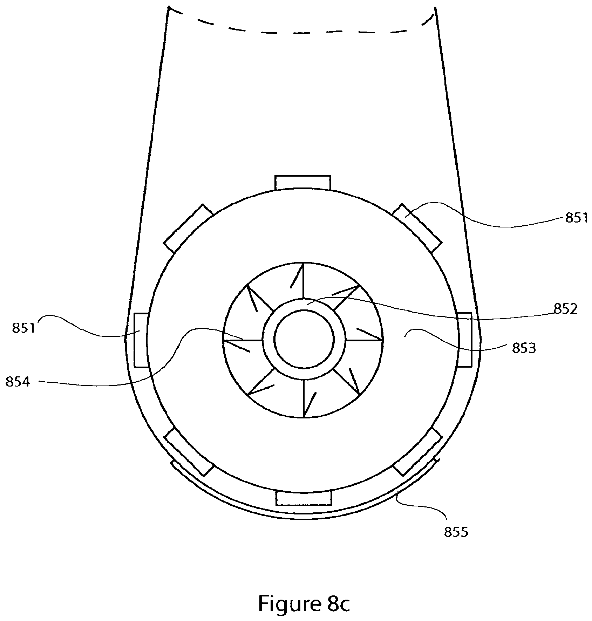

In a further embodiment, illustrated in Figure 8c, an ultrasonic rotary motor is provided, where the rotor of the motor directly supports a ring array of transducers. 3 As shown in Figure 8c, there is an ultrasonic motor consisting of a rotor 853 supporting the ultrasound transducers 851. The rotor surrounds a piezoelectric ring resonator 852. The piezoelectric resonator is connected to steel pushers 854. A standing ultrasonic wave is induced in the resonator 852 causing the ring to expand and contract in a radial direction. The pushers vibrate in a manner resulting in elliptical movement of the pushers. The pushers are held against the rotor 853, causing the rotor to rotate by frictional engagement between the rotor and the pushers.

This rotary motor drives the transducers 851 in a circle, past ultrasound window 855. Each ultrasound transducer is activated only when the transducer is aligned with the ultrasound window, and thus in a position to insonify a portion of the target to be imaged.

Ultrasonic motor efficiency is insensitive to size and hence they are superior in the mini-motor area. This allows for the construction of devices of small size and low weight.

Ultrasonic motors do not produce the electromagnetic interference which is inherent in the operation of electromagnetic motors. This is an advantage for ultrasound systems where the electrical signals being received are inherently of low power and susceptible to interference. This has a further advantage for hand held ultrasound systems in reducing the need for shielding between the electronic components and the motor, which reduces size, weight and cost.

In order to produce ultrasound energy for scanning, the ultrasound transducer elements are stimulated by a high voltage power supply. This high power supply would generally supply power at voltage magnitudes on the order of tens or hundreds of volts. The ultrasonic elements which form the ultrasonic motors in those embodiments which employ ultrasonic motors are also driven by high voltage power supplies which may have similar output characteristics. In further embodiments, the ultrasound transducer elements and the ultrasonic motor are driven from a common power supply or power supplies. This results in lower costs.

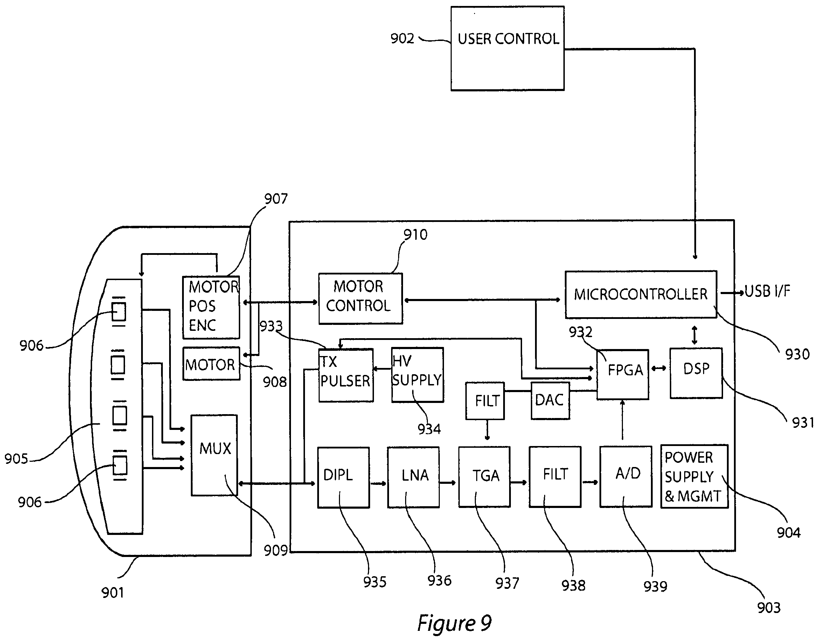

In Figure 9, there is shown an architecture of an embodiment of the present invention. The block diagram shows a probe unit, including a scan head 901 and a probe unit body 903. There may also be a host unit (not illustrated) providing a display and at least part of a user interface, connected to the system using a USB interface 920.

Small size and weight are desirable for the probe unit to facilitate hand held use. In an embodiment, the size of the probe unit is about 15cmx8cmx3cm. In a further embodiment, the weight of the probe unit is 500g or less. In a preferred embodiment the size of the probe unit is about 11 cmx6cmx2cm and the weight is about 200g.

The scan head 901 includes transducers 906, arranged as a transducer array on a slider element 905. The slider element is driven by ultrasonic motor 908. The position of the slider 905 is monitored by position encoder 907.

The transducers 906 are connected to multiplexer 909. Electrical signals to excite the transducers to produce ultrasound output, and the electrical signals from the transducers in response to received echoes, are passed through the multiplexer 909. The multiplexer may be provided within the probe body 903, or omitted, at the expense of providing more connections between the scan head 901 and the probe unit body 903.

User control of the ultrasound scanning system is via a user interface which may be provided wholly on the host unit, wholly on the probe unit, or split or duplicated between the probe unit and the host unit. In the illustrated embodiment there is a User Control Panel 902 incorporated in the probe unit, said Panel including a freeze button to start and stop scanning, a set of buttons providing increment, decrement and select functionality, and a "back" button. These buttons are used for basic control of the system, including without limitation, some or all of start and stop scan, depth adjustments, gain adjustments, operating mode selections, and preset selections as well as other basic settings. The user control panel 902 sends data identifying the state of the buttons to a micro-Controller 930, which in this embodiment is part of a combined microcontroller/DSP device such as the OMAP3525 Applications Processor from Texas Instruments. The microcontroller monitors the state of the control panel and provides appropriate commands to control the appropriate electronic circuitry to allow the user to control the ultrasound system. Where this requires a graphical user interface, this is displayed on the host unit.

The microcontroller 930 contains a set of parameters for controlling the operation of the device during scanning. At initial power up, a default set of parameters are created, which can then be modified by the user before or during scanning. The parameters include without limitation, the operating frequency, the active scanning mode, the gain curve, the scanning depth, the Doppler gate depth and angle if required, color Doppler ranges if required, power Doppler ranges if required, and M-mode pulse repetition rate. The scanning modes available may include, without limitation any or all of B-mode, M-mode, and modes available using Pulse Wave Doppler, including color Doppler, power Doppler and spectral Doppler, and Duplex Doppler.

The microcontroller passes the relevant parameters to the Digital Signal Processor (DSP) 931 and Field Programmable Gate Array (FPGA) 932 when scanning is activated or the parameters change.

When a user commences a scan, either by pressing a button on the control panel or by activating a control on the host, the microcontroller sends a command to the DSP and Field Programmable Gate Array (FPGA) to activate scanning, along with any updated configuration parameters. The DSP is configured to receive and process ultrasound data according to the parameters, which may include parameters concerning Doppler processing.

The microcontroller and the FPGA together provide the functionality to control the scan head ultrasonic motor. The ultrasonic motor position encoder 908 produces a value proportional to the position of the ultrasonic motor at any point in time. This position is saved with a timestamp in a register in the FPGA, and the microcontroller reads this information to calculate a velocity. The velocity is compared with the desired velocity. The motor control unit 910 is instructed to alter the voltage and frequency of the drive signal applied to the ultrasonic motor 908 in order to achieve the desired velocity.

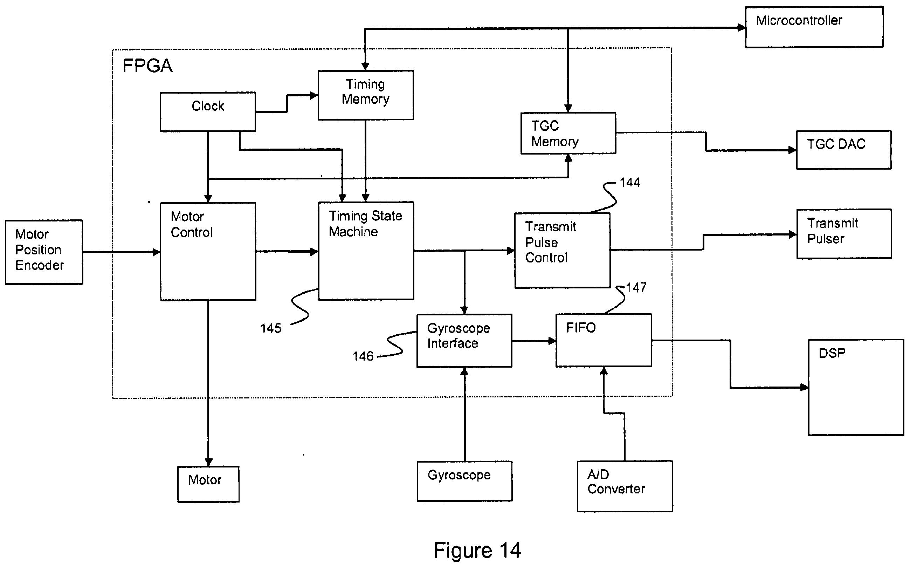

Figure 14 illustrates the FPGA functionality. The FPGA receives and decodes the output of the ultrasonic motor position encoder 907, and provides the information to the microcontroller in order for it to recalculate the ultrasonic motor drive signals. The FPGA includes a timing state machine 145 which uses the decoded position information to determine the appropriate time to generate the next ultrasound pulse sequence to be output from the transducers, which is achieved by driving the transmit pulse controller 144.

Transmit pulse controller 144 generates control signals corresponding to the type of scan line required to be generated. For a B-mode scan line, a single pulse is generated at the imaging frequency at the voltages provided by the High Voltage power supply (HV Supply) 934. These voltages are typically up to +-100V. For Doppler a sequence of several pulses, typically eight pulses, at the Doppler imaging frequency is generated. These Doppler pulses are typically of longer duration than B-mode pulses. The multiplexer is preconfigured before the Tx Pulser 933 fires to steer the transmit pulse to the appropriate circular transducer crystal. The transducer crystal 906 generates an ultrasonic waveform in response to the electrical pulse, which is transmitted into a body to be imaged.

The ultrasonic waveform is reflected by changes in acoustic impedance, producing echoes which are received back at the transducer crystal 906 at lower pressure. The crystal converts this lower pressure waveform into an electrical signal, which is directed through the multiplexer to the input protection circuitry 935. The input protection circuitry protects the sensitive low noise amplified (LNA) 936 from the high voltage transmit pulse while letting through a low voltage receive signal. Several input protection circuits are known in the prior art.

The low noise amplifier (LNA) provides amplification of the small receive signal, while adding little noise to the output signal. The LNA is typically single ended, and generates a differential output voltage for feed into a time gain amplifier (TGA) 937. The TGA provides further amplification with the amplification provided being dependent on time. Ultrasound signals attenuate as they propagate through tissue, and the TGA compensates for this attenuation by increasing the gain dependent on the time from the beginning of the received pulse, which is proportional to the depth of the echo reflection.

The output of the time gain amplifier is filtered to remove as much noise as possible and to prevent aliasing. Typically a bandpass filter 938 is used. The output of the filter is input to the analog to digital converter 939. A sampling frequency of at least 4x the imaging frequency is preferred. The analog to digital output is input to a first in first out memory (FIFO) 147 in the FPGA 932. The FPGA adds some header information to each complete scan line, including the type of scan line (for example, B-mode or Doppler), the motor positional encoder input if required, and a time count. This information may be used at a later stage in processing dependent on the configuration parameters.

The DSP reads the scan lines out of the FPGA FIFO, and performs appropriate processing on the data depending on the configuration parameters.

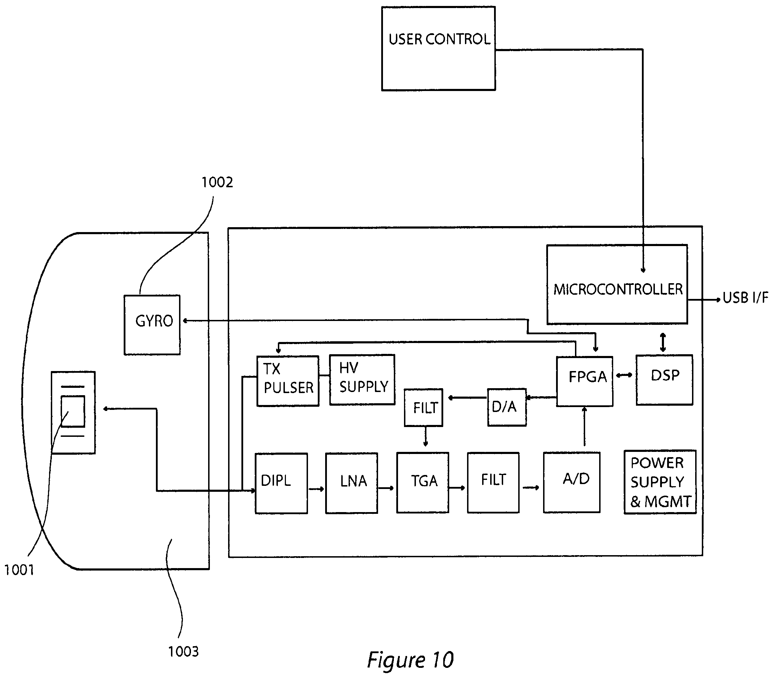

Figure 10 is a configuration of the system which does not have real-time ultrasound capability, but uses a lower cost single channel system where the ultrasound beam is stationary with respect to the system. A single transducer 1001 is provided, which is in a fixed relationship to the probe unit body. There is also provided a gyroscope 1002. Images created by moving the probe unit in an arc with the point of contact of the scan head 1003 with the patient being substantially constant while the transducer is pulsed. This will generate a sequence of scan lines which together make up a sector image. The gyroscope provides information as to the relative position of each scan line, enabling the scan lines to be assembled into an image.

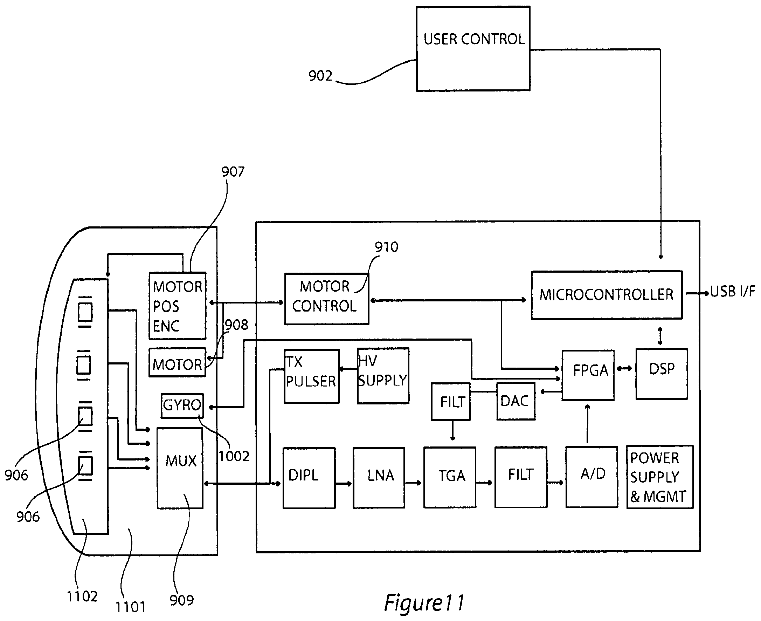

Figure 11 illustrates a further embodiment of the invention, where the scan head 1101 contains a motor 908 and a gyroscope 1103. The gyroscope is oriented so it provides angular measurements perpendicular to the image plane of the B-mode image which is produced from the transducer array 1102. By providing the gyroscope, the system is able to construct 3D images from a sequence of 2D image scans, and determine volumes. In use, a series of B-mode images are made as for the system of Figure 9. The probe unit is moved in an arc perpendicular to the image plane of the B-mode images. The operation is otherwise similar to the system as described in Figure 9.

Referring to Figure 14, The FPGA timing state machine 145 interfaces to the gyroscope in embodiments where a gyroscope is provided. The gyroscope angular measurement is combined with the ultrasound scan line information in a first in first out (FIFO) memory 147, and read by the digital signal processor (DSP). Where a single transducer is used, the gyroscope information is used to assemble the scan lines to form a sector image. Where a transducer array is used, the gyroscope information is used to assemble B-mode sector images into a representation of a volume which may also be used for any applications which require 3D volume calculations.

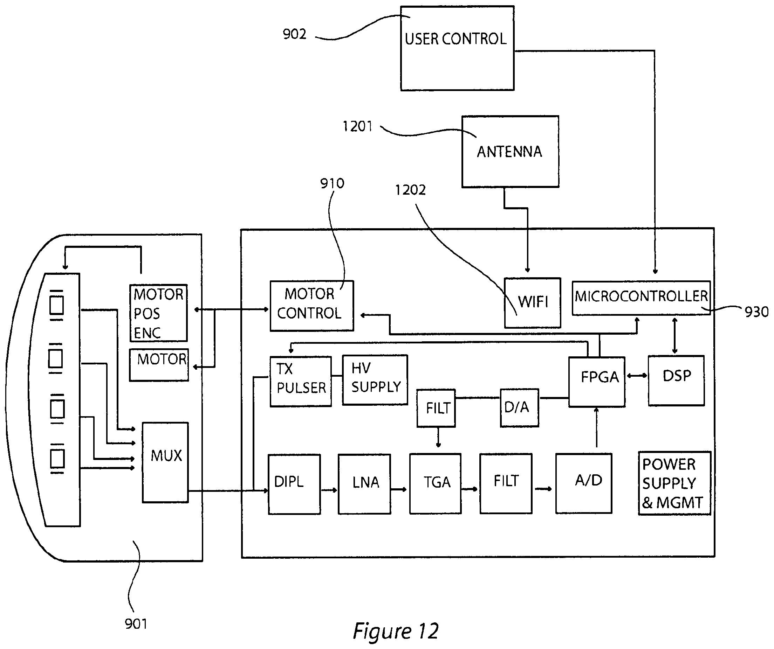

Figure 12 illustrates an alternative embodiment of the invention, whereby the interface to the host display and control system is via a wireless interface. There is provided a wireless interface module 1202 controlled by the microcontroller 930. An antenna 1201 is provided for signal transmission. This interface provides communication to and from the host unit. No USB interface is required for communication with the host unit, although one may be provided for access to third party hardware. In a presently preferred embodiment the wireless protocol used is WiFi Direct.

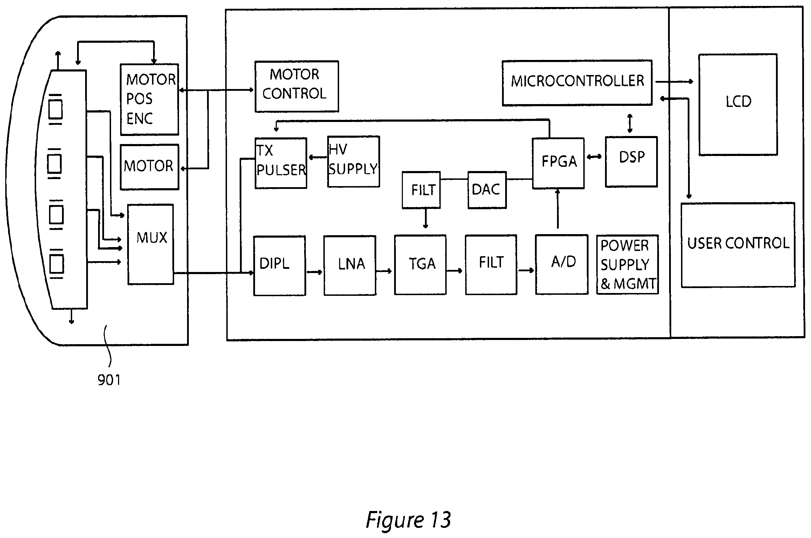

Figure 13 illustrates an alternative embodiment of the invention, wherein there is no host unit. There is provided a display 1301 which is integral with the probe unit.

There is also provided an enhanced user control module 1302. This includes the functionality of the control panel of previously described embodiments, but further includes functionality allowing for full control of the system in the absence of a host unit. The control module may include a graphical user interface displayed on the display 1301. The display may be a touchscreen, able to provide input to the user interface.

The operation of the DSP in different embodiments will vary. Where a host control and display system is connected, some processing and control functions and the display function may be performed by the host unit. Where no host is used, all functions are performed by the probe unit, increasing the load on the DSP. Limitations on the division of functionality between a host and the probe unit come from the processing power of the host and the bandwidth available for transmission between the host and the probe unit. In general, the implementation and subdivision of algorithms is designed to minimise the host processing requirements and the bandwidth required for transmission.

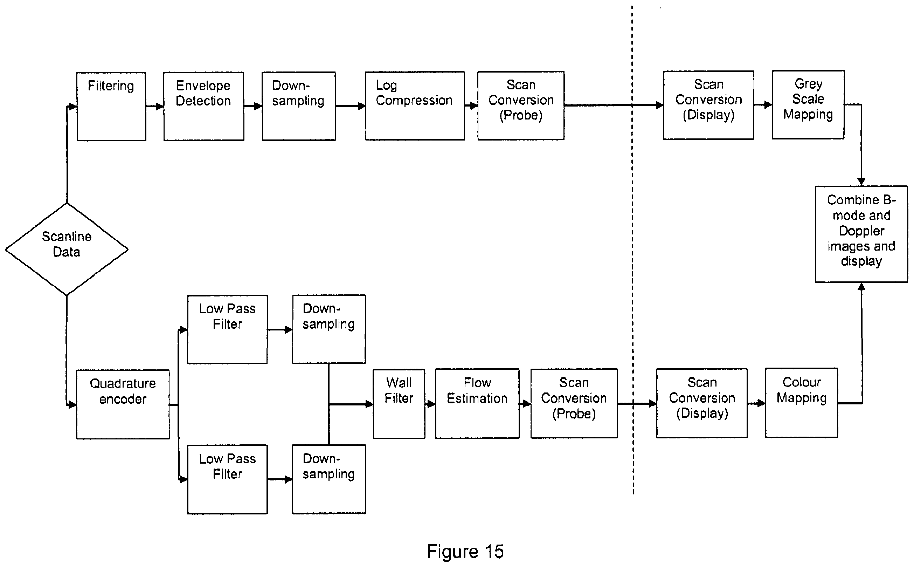

Figure 15 illustrates the algorithms for processing combined B-mode/Doppler, with a division of the tasks between the ultrasound probe unit and the host display system.

The first step in the B-Mode processing chain is to filter the digitised incoming rf scan line data using an FIR bandpass filter. The filter is adjusted depending on the transducer connected to the system and the imaging frequency. For example, for a 3MHz imaging frequency a bandpass filter of 1 to 5MHz could be used. For an 8MHz imaging frequency a bandpass filter of 4.5 to 11.5MHz could be used. Following filtering, the envelope of the rf scan line data is generated. The preferred method is to use a Hilbert transform to generate the in-phase (I) and quadrature (Q) components of the rf scan line. The final envelope is generated by summing the squares of the I and Q components, and taking the square root of the result.

$$Envelope = \sqrt{I^2 + Q^2}$$

There are a number of algorithms for generating Hilbert Transforms. The preferred embodiment is using an FIR approximation.

As part of the envelope generation, the scan line can be downsampled or decimated. Downsampling by a factor of 2-4 is possible, depending on the scan conversion algorithm used. In the preferred embodiment, the scan line is downsampled by a factor of 4 with scan conversion using bilinear interpolation. An alternative is to downsample by a factor of 2 and use a less computationally intensive interpolation algorithm, such as one which computes pixel intensity from the average of sample points inside the pixel area, and interpolates for other pixels between adjacent pixels.

Following downsampling, the scan line is compressed from the analog to digital word size into an 8 bit word size to map the signal into the desired grey scale levels for display.

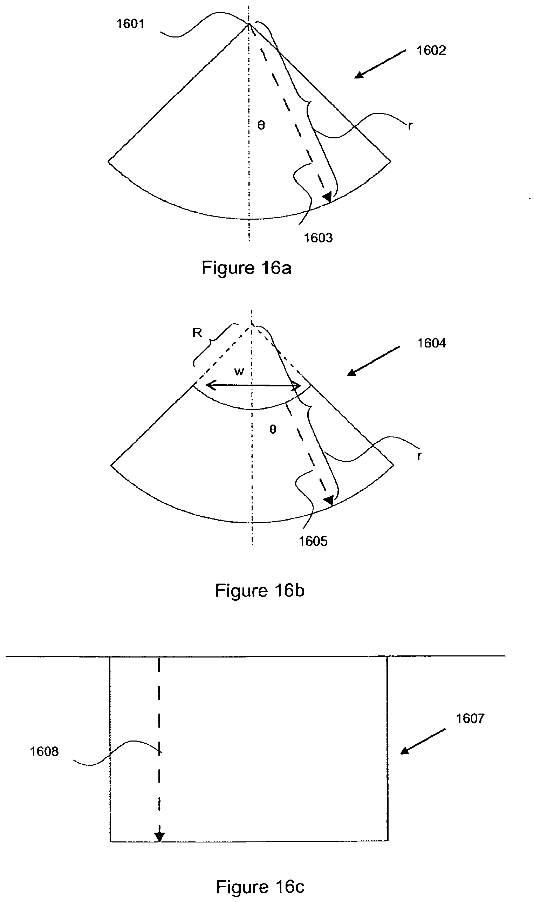

The downsampled scan lines are then converted to a rectangular image display through scan conversion. The scan conversion is performed in 2 stages. The first stage is converting the image into a compressed rectangular array with high resolution, preferably with pixel resolution of less than half the axial resolution of the ultrasound pulse. A common scan conversion algorithm is a 2x2 bilinear interpolation, mapping the scan line points from a polar co-ordinate system to a rectangular co-ordinate system. Referring to Figure 16a there is shown an idealised scan from a phased array or small diameter ring transducer. The scan area 1602 can be seen as a sector of a circle, with origin 1601. In practice, the transducer is not a point source, but it is small compared to the depth of scan. The location of each scanline 1603 can be characterised in polar co-ordinates by a length r and an angle $\theta$. Referring to Figure 16b, there is shown a sector scan 1604 for a curvilinear array with a radius of curvature R and a width W. The scan is a truncated sector of a circle. Each scanline 1605 can be characterised by polar co-ordinates of a length R+r and an angle θ.

Figure 16c shows an idealised scan 1607 for a linear array, with scanline 1608. The characterising co-ordinates are linear, but in general will not correspond to the linear co-ordinate system desired for display.

In each of the case illustrated in Figure 16, scan conversion is required to convert the acquired scanline data into pixel data for display of the image to a user.

For sector shaped images, the compression algorithm reduces the image size by ignoring image area which is not containing actual image data. Several methods are possible to perform this, with one being a simple format where each pixel row contains a header with the starting pixel and number of pixels with valid data. This technique yields a compression ratio of close to 2. Another lossless technique which would provide reasonable results is run-length encoding. LZW encoding, or Huffman based encoding such as png could be used.

The high resolution compressed rectangular array is handled differently depending on the system configuration. The array is stored in a local memory, with typically up to 100 compressed frames being stored locally. At the same time the current frame is transmitted from the ultrasound probe to a host display system. The host display system completes the processing by decompressing the image into a high resolution buffer, interpolating from the high resolution buffer to an interim display image buffer, applying any grey scale adjustment, combining with any Doppler image information if required, and finally writing to the display buffer.

Figure 15 also illustrates the steps required for processing and displaying Doppler information overlayed on the B-mode image. When generating Doppler information, the system generates a sequence of Doppler pulses with a consistent phase and processes the received set of scan lines. The raw input rf scanlines are quadrature encoded, with the inphase (I) and quadarture (Q) components extracted by multiplying the echo signal by the cosine and sine of the transducer excitation frequency. The I and Q outputs from the quadrature encoder are low pass filtered and decimated or downsampled. A downsampling ratio of four produces satisfactory results, but other factors may be employed. The output of the downsampler is saved into a buffer until a complete set of scan lines is received. Typically a set will be eight scan lines, although the size of the set can be adjusted. The data set is filtered using a Wall filter. The function of the wall filter is to reduce contribution from large, slow moving features such as an abdominal wall.

The wall filter in the illustrated embodiment is an FIR type high pass filter. An alternative is to use a state-space formulation IIR filter, which reduces the transient response length of the IIR filter. The state-space initialisation provides satisfactory attenuation using a step initialisation scheme.

In the preferred embodiment the filtered set of scan lines is processed using autocorrelation techniques to generate velocity, power, and turbulence information. The Doppler power of the set of scan lines is calculated as follows:

$$P_d = \sum_{k=0}^{n-2} s_{d,k} \cdot s_{d,k}^*$$

The correlation between adjacent scan line points is calculated as follows:

$$c_d = \sum_{k=0}^{n-2} s_{d,k+1} \cdot s_{d,k}^*$$

The velocity estimation is calculated as follows:

$$v_d = tan^{-1}\left(\frac{Im(c_d)}{Re(c_d)}\right)$$

Turbulence estimation is calculated as follows:

$$t_d = 1 - \left(\frac{|c_d|}{P_d}\right)$$

where

$s$    is the complex representation of a scan line ($s = I + j Q$)

$d$    is the sample at a depth in a scan line.

k    is the scan line number in a set of scan lines.

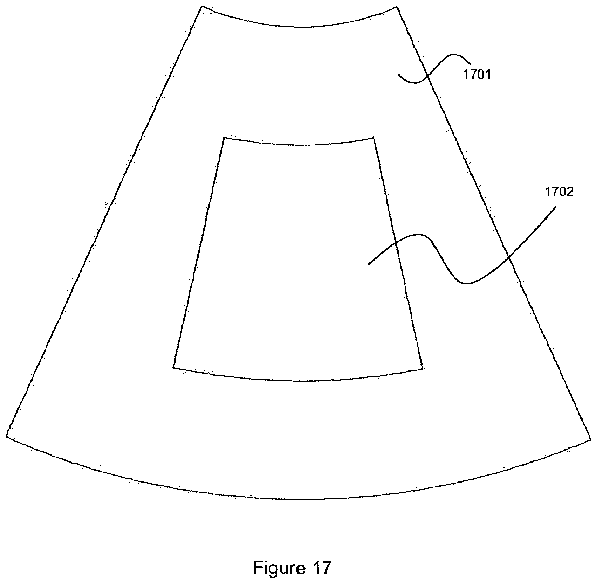

When the system is configured for power Doppler, only the power estimation is required. The correlation between adjacent scan lines, velocity, and turbulence are not required. When the system is configured for color Doppler, the system calculates velocity, and optionally power and turbulence. The output of the flow and turbulence estimation is scan converted to a rectangular array, of size 200 x 200 x 8 for each flow estimator in this embodiment, other sizes are possible. The rectangular arrays are compressed using a simple lossless compression algorithm. Figure 17 provides an illustration of a typical Doppler window 1702, where the Doppler processing area is limited with respect to the full B-mode image 1701 size. This improves the compression of the 200x200x8 arrays, as a large proportion of the array is empty. The compressed array are transmitted to the host, with a further interpolation step to the required Doppler image window, and color map conversion. Positive velocities are encoded in red shades, while negative velocities are encoded in blue shades. Turbulence is encoded in green. When power Doppler is selected, only red shades are used to represent the total power of the Doppler signals.

An alternative embodiment of the invention is to provide a system which plots power Doppler with different color schemes used depending on whether the direction of flow is positive or negative. The power Doppler is calculated as above, scan converted, and transmitted to the host. In parallel, an efficient method is used to determine the direction of the flow. The full correlation between adjacent scan line points can be used, with the sign of the real and imaginary outputs used for each point to determine a direction. Alternatively, for imaging where sensitivity is not an issue, the correlation to decode direction can be shortened, and use a smaller set of scan lines. Power is optimised by providing a system which has feedback, where a small set of scan lines is used, and if the direction result is unstable (changing rapidly) the number of scan lines is increased to maximum. Then when the result is stable, the system runs a test correlation in parallel on a smaller set of scan lines and if that result is stable moves to using the smaller set. The system therefore optimises the power transmitted into the body, and minimises power consumption.

The system supports Cine playback or video recording. For Cine playback, the ultrasound probe stores compressed 500x500x8 B-mode frames into local DSP memory. 100 frames provides around five seconds of automatic Cine recording. When the user instructs the host user interface to scroll back through recorded frames, the DSP transmits the compressed frames up to the host and scan conversion is completed as per normal operation. For video recording, when the user instructs the host to record a segment to video, the DSP activates operations to complete any scan conversion and Doppler combining, and runs a video encoder which records the real-time images to a video format saving directly to memory, preferably at 640x480 pixel resolution. The video file can then be played back by a video decoder streaming data to a frame buffer which is transmitted to the host for display.

The system can be configured to operate in M-mode or duplex mode where a B-mode image and M-mode image are shown on the same screen.

In M-mode or Duplex mode, the DSP reads the M-mode scan lines and interpolates each scan line to a 500x1 x8 buffer. The M-mode buffer is transmitted to the host which performs grey scale adjustment and renders the scan line to the display.

For duplex mode, two sorts of M-mode are possible. Full duplex is where an M-mode scan line is placed on a realtime B-mode image, and the M-mode pulse repetition rate is the same rate as the B-mode image update rate, typically 20 frames per second.

For quasi duplex mode, a high M-mode frame rate is required, such as for looking at rapidly moving heart valves. In this mode, an M-mode line is placed on a B-mode image, and when the select button is pressed the B-mode image is frozen and the M-mode graph is rendered to the screen.

A quasi duplex gated Doppler mode is also provided. A Doppler line is placed by the user on a B-mode image, and a gate area moved along the line to select the area for Doppler analysis. The gate angle can also be adjusted. When the select button is activated, the B-mode image is frozen, and a spectral Doppler graph - which displays time, frequency and magnitude of the Doppler shift - is plotted. The Doppler signal is processed in a similar way to color Doppler, except the scanlines are repeatedly pulsed only along the selected line (rather than moving across the image). In addition, the user selects a particular depth of interest, and the received echoes are 'range gated' such that only Doppler information from said depth of interest is analysed. This provides a large number of scanlines from the region of interest; frequency analysis is carried out on large groups of these scanlines (say 256) at a time, with the preferred method being applying the Fast Fourier Transform (FFT) on the complex representation of a group of scanlines. Each FFT performed provides a frequency spectrum of the Doppler signal shift, and these spectra can be displayed together to generate a time-frequency plot of the flow characteristics at the depth of interest which is often called 'spectral Doppler'. Spectral Doppler enables visualisation of how the velocity and power of blood flow changes with time for the selected region of interest.

**Claims**

1. A handheld ultrasound imaging apparatus (300) adapted to produce ultrasound images of a selected lateral resolution;
   wherein the apparatus (300) includes an array of ultrasound transducers (404, 502-505, 605-608, 615, 618, 701, 817, 906, 1102) attached to a translating element (402, 501, 600, 700, 730, 805, 818), the translating element being adapted to be mechanically moved in a repetitive motion a selected linear distance along a linear path, the apparatus being operable to acquire a plurality of scanlines during each motion repetition, the distance between adjacent transducers (404, 502-505, 605-608, 615, 618, 701, 817, 906, 1102) being greater than the minimum separation of adjacent scanlines required to produce an ultrasound image of the selected lateral resolution;
   **characterised in that**:

   at least one of the transducers (404, 502-505, 605-608, 615, 618, 701, 817, 906, 1102) is adapted to acquire, during the repetitive motion, B-mode scanline data and Doppler scanline data such that a plurality of information displays having separately selected lateral resolutions are produced concurrently, the plurality of information displays including B-mode imaging and at least one of Gated Doppler, Power Doppler, Pulsed Wave Doppler, Colour Doppler, Duplex Doppler and M-mode imaging, and wherein said handheld ultrasound transducer imaging apparatus (300) is operable so that Doppler scanline data is acquired only when the array is stationary, and B-mode scanline data is continuously acquired when the array is in motion.

2. The apparatus of claim 1 further **characterised in that** the distance between adjacent transducers (404, 502-505, 605-608, 615, 618, 701, 817, 906, 1102) attached to the translating element (402, 501, 600, 700, 730, 805, 818), along the linear path is of approximately the same magnitude as the selected linear distance.

3. The apparatus of any one of the preceding claims further **characterised in that** the translating element (402, 501, 600, 700, 730, 805, 818), is driven by an ultrasonic motor.

4. The apparatus of any one of the preceding claims further **characterised in that** the translating element has a curvilinear shape and the linear path is a curvilinear path.

5. The apparatus of any one of the preceding claims further **characterised in that** the movement of the translating element (402, 501, 600, 700, 730, 805, 818), is able to be controlled to stop at a selected stop position during subsequent motion repetitions with an error of less than 500 nanometres.

6. A method of producing an ultrasound image comprising the steps of:

providing a plurality of ultrasound transducers (404, 502-505, 605-608, 615, 618, 701, 817, 906, 1102) arranged in an array,
acquiring at least one ultrasound scanline by firing at least one of the transducers (404, 502-505, 605-608, 615, 618, 701, 817, 906, 1102) and receiving ultrasound echoes returned from a body to be imaged,
moving the array an incremental distance,
acquiring further ultrasound scanlines from the transducers,
repeating the steps of acquiring scanlines, and moving the array until the array has moved a selected distance, the selected distance being substantially greater than the incremental distance,
displaying the acquired scanlines in a spatial relationship proportional to the spatial relationship in which the scanlines were captured in order to display an ultrasound image,
**characterised in that**
B-mode scanline data and Doppler scanline data are acquired such that a plurality of information displays having separately selected lateral resolutions are produced concurrently, the plurality of information displays including B-mode imaging and at least one of Gated Doppler, Power Doppler, Pulsed Wave Doppler, Colour Doppler, Duplex Doppler, and wherein Doppler scanline data is acquired only when the array is stationary, and B-mode scanline data is continuously acquired when the array is moving.

7. The method of claim 6 wherein the array is moved in a series of discrete steps.

8. The method of claim 6 wherein the movement of the array and the acquiring of scanline data are substantially continuous between start and stop commands from a user.

**Patentansprüche**

1. Handgehaltene Ultraschall-Bildgebungsvorrichtung (300), die dazu ausgelegt ist, Ultraschallbilder mit einer ausgewählten seitlichen Auflösung zu erzeugen;
wobei die Vorrichtung (300) eine Anordnung von Ultraschalltransducern (404, 502-505, 605-608, 615, 618, 701, 817, 906, 1102), die an einem Übersetzungselement (402, 501, 600, 700, 730, 805, 818) angebracht sind, enthält, wobei das Übersetzungselement dazu ausgelegt ist, einen ausgewählten linienförmigen Abstand entlang eines linienförmigen Pfades in einer sich wiederholenden Bewegung mechanisch bewegt zu werden, wobei die Vorrichtung dazu dient, mehrere Scanlinien während jeder Bewegungswiederholung zu erfassen, wobei der Abstand zwischen benachbarten Transducern (404, 502-505, 605-608, 615, 618, 701, 817, 906, 1102) größer als die minimale Trennung benachbarter Scanlinien, die zur Erzeugung eines Ultraschallbildes der ausgewählten seitlichen Auflösung erforderlich sind, ist;
**gekennzeichnet dadurch, dass**:

mindestens einer der Transducer (404, 502-505, 605-608, 615, 618, 701, 906, 1102) dazu ausgelegt ist, während der wiederkehrenden Bewegung B-Mode-Scanliniendaten und Doppler-Scanliniendaten zu erfassen, so dass mehrere Informationsanzeigen mit getrennt ausgewählten seitlichen Auflösungen zugleich erzeugt werden, wobei die mehreren Informationsanzeigen B-Mode-Bildgebung und mindestens eines von Gated-Doppler-, Power-Doppler-, Pulswellen-Doppler-, Farb-Doppler-, Duplex-Doppler- und M-Mode-Bildgebung enthalten und wobei die handgehaltene Ultraschalltransducer-Bildgebungsvorrichtung (300) dazu

dient, Doppler-Scanliniendaten nur zu erfassen, wenn die Anordnung im stationären Zustand ist, und B-Mode-Scanliniendaten kontinuierlich zu erfassen, wenn die Anordnung in Bewegung ist.

2. Vorrichtung nach Anspruch 1, ferner **dadurch gekennzeichnet, dass** der Abstand zwischen benachbarten Transducern (404, 502-505, 605-608, 615, 618, 701, 817, 906, 1102), die am Übersetzungelement (402, 501, 600, 700, 730, 805, 818) angebracht sind, entlang des linienförmigen Pfades ungefähr von der gleichen Größenordnung wie der ausgewählte linienförmige Abstand ist.

3. Vorrichtung nach einem der vorhergehenden Ansprüche, ferner **dadurch gekennzeichnet, dass** das Übersetzungselement (402, 501, 600, 700, 730, 805, 818) von einem Ultraschallmotor angetrieben wird.

4. Vorrichtung nach einem der vorhergehenden Ansprüche, ferner **dadurch gekennzeichnet, dass** das Übersetzungselement eine krummlinige Form aufweist und der linienförmige Pfad ein krummliniger Pfad ist.

5. Vorrichtung nach einem der vorhergehenden Ansprüche, ferner **dadurch gekennzeichnet, dass** die Bewegung des Übersetzungselements (402, 501, 600, 700, 730, 805, 818) so gesteuert werden kann, dass es an einer ausgewählten Anhalteposition während aufeinander folgender Bewegungswiederholungen mit einem Fehler von weniger als 500 Nanometern anhält.

6. Verfahren zum erzeugen eines Ultraschallbildes, umfassend die folgenden Schritte:

Vorsehen mehrerer in einer Anordnung angeordneter Ultraschalltransducer (404, 502-505, 605-608, 615, 618, 701, 817, 906, 1102),
Erfassen mindestens einer Ultraschallscanlinie durch Ansteuern mindestens eines der Transducer (404, 502-505, 605-608, 615, 618, 701, 817, 906, 1102) und
Empfangen von Ultraschallechos, die von einem der Bildgebung unterzogenen Körper zurückkehren,
Bewegen der Anordnung mit einem zunehmenden Abstand,
Erfassen weiterer Ultraschallscanlinien von den Transducern,
Wiederholen der Schritte des Erfassens von Scanlinien, und
Bewegen der Anordnung, bis die Anordnung einen ausgewählten Abstand bewegt worden ist, wobei der ausgewählte Abstand im wesentlichen größer als der zunehmende Abstand ist,

Anzeigen der erfassten Scanlinien in einer räumlichen Beziehung, die proportional zu der räumlichen Beziehung ist, in der die Scanlinien erfasst worden sind, um ein Ultraschallbild anzuzeigen,
**gekennzeichnet dadurch, dass**
B-Mode-Scanliniendaten und Doppler-Scanliniendaten so erfasst werden, dass mehrere Informationsanzeigen mit getrennt ausgewählten seitlichen Auflösungen zugleich erzeugt werden, wobei die mehreren Informationsanzeigen B-Mode-Bildgebung und mindestens eines von Gated-Doppler-, Power-Doppler-, Pulswellen-Doppler-, Farb-Doppler-, Duplex-Doppler-Bildgebung enthalten und wobei Doppler-Scanliniendaten nur erfasst werden, wenn die Anordnung im stationären Zustand ist, und B-Mode-Scanliniendaten kontinuierlich erfasst werden, wenn sich die Anordnung bewegt.

7. Verfahren nach Anspruch 6, wobei die Anordnung in einer Reihe diskreter Schritte bewegt wird.

8. Verfahren nach Anspruch 6, wobei die Bewegung der Anordnung und das Erfassen von Scanliniendaten im wesentlichen kontinuierlich zwischen Start- und Stop-Anweisungen von einem Benutzer stattfinden.

## Revendications

1. Appareil d'imagerie ultrasonore portable (300) adapté pour produire des images ultrasonores à résolution latérale sélectionnée ;
dans lequel
l'appareil (300) comprend un groupe de transducteurs ultrasonores (404, 502-505, 605-608, 615, 618, 701, 817, 906, 1102) reliés à un élément de translation (402, 501, 600, 700, 730, 805, 818), l'élément de translation étant adapté pour être déplacé mécaniquement selon un mouvement répétitif sur une distance linéaire sélectionnée le long d'une trajectoire linéaire, l'appareil pouvant être utilisé pour acquérir une pluralité de lignes de balayage durant chaque répétition de mouvement, la distance entre les transducteurs adjacents (404, 502-505, 605-608, 615, 618, 701, 817, 906, 1102) étant supérieure à la séparation minimum des lignes de balayage adjacentes requise pour produire une image ultrasonore à résolution latérale sélectionnée ;
**caractérisé en ce que** :

au moins l'un des transducteurs (404, 502-505, 605-608, 615, 618, 701, 817, 906, 1102) est adapté pour acquérir, durant le mouvement répétitif, des données de lignes de balayage en mode B et des données de lignes de balayage Doppler de sorte que plusieurs affichages d'informations ayant des résolutions latérales sélectionnées de façon séparée soit produits en même temps, la pluralité d'affichages d'informations comprenant une imagerie en mode B et au moins l'un d'un Doppler à fenêtre, d'un Doppler de puissance, d'un Doppler à ondes pulsées, d'un Doppler en couleur, d'un Doppler duplex, et d'une imagerie en mode M, et dans lequel ledit appareil d'imagerie ultrasonore portable (300) peut fonctionner de sorte que les données de lignes de balayage Doppler soient acquises uniquement lorsque le groupe est stationnaire, et que les données de lignes de balayage en mode B soient acquises en continu lorsque le groupe est en mouvement.

2. Appareil selon la revendication 1, **caractérisé en outre en ce que** la distance entre les transducteurs adjacents (404, 502-505, 605-608, 615, 618, 701, 817, 906, 1102) reliés à l'élément de translation (402, 501, 600, 700, 730, 805, 818), le long de la trajectoire linéaire, présente approximativement la même magnitude que la distance linéaire sélectionnée.

3. Appareil selon l'une quelconque des revendications précédentes, **caractérisé en outre en ce que** l'élément de translation (402, 501, 600, 700, 730, 805, 818) est entraîné par un moteur ultrasonore.

4. Appareil selon l'une quelconque des revendications précédentes, **caractérisé en outre en ce que** l'élément de translation possède une forme curviligne, et la trajectoire linéaire est une trajectoire curviligne.

5. Appareil selon l'une quelconque des revendications précédentes, **caractérisé en outre en ce que** le mouvement de l'élément de translation (402, 501, 600, 700, 730, 805, 818) peut être contrôlé pour s'arrêter à une position d'arrêt sélectionnée durant les répétitions de mouvement ultérieures, avec une erreur inférieure à 500 nanomètres.

6. Procédé de production d'imagerie ultrasonore comprenant les étapes qui consistent à :

prévoir une pluralité de transducteurs ultrasonores (404, 502-505, 605-608, 615, 618, 701, 817, 906, 1102) disposés en groupe,
acquérir au moins une ligne de balayage ultrasonore en déclenchant au moins l'un des transducteurs (404, 502-505, 605-608, 615, 618, 701, 817, 906, 1102), et
recevoir les échos ultrasonores renvoyés par un corps à imager,
déplacer le groupe sur une distance incrémentale,
acquérir d'autres lignes de balayage ultrasonores depuis les transducteurs,
répéter les étapes d'acquisition des lignes de balayage, et
déplacer le groupe jusqu'à ce que le groupe se soit déplacé sur une distance sélectionnée, la distance sélectionnée étant sensiblement supérieure à la distance incrémentale,
afficher les lignes de balayage acquises selon une relation spatiale proportionnelle à la relation spatiale selon laquelle les lignes de balayage ont été capturées, afin d'afficher une image ultrasonore,
**caractérisé en ce que**
les données de lignes de balayage en mode B et les données de lignes de balayage Doppler sont acquises de sorte que plusieurs affichages d'informations ayant des résolutions latérales sélectionnées de manière séparée soient produits en même temps, la pluralité d'affichages d'informations comprenant l'imagerie en mode B et au moins l'un d'un Doppler à fenêtre, d'un Doppler de puissance, d'un Doppler à ondes pulsées, d'un Doppler en couleur, d'un Doppler duplex, et dans lequel les données de lignes de balayage Doppler sont acquises uniquement lorsque le groupe est stationnaire, et les données de lignes de balayage en mode B sont acquises en continu lorsque le groupe est en mouvement.

7. Procédé selon la revendication 6, dans lequel le groupe est déplacé selon une série d'étapes discrètes.

8. Procédé selon la revendication 6, dans lequel le mouvement du groupe et l'acquisition des données des lignes de balayage sont sensiblement continus entre la commande de démarrage et la commande d'arrêt provenant d'un utilisateur.

Prior Art

Figure 1a

Prior Art

Near
Field

Far Field

Figure 1b

EP 2 426 509 B1

Prior Art

202

201

203

Figure 2a

Prior Art

202

205

203

204

Figure 2b

19

*Figure 3*

EP 2 426 509 B1

*Figure 4*

Figure 5a

EP 2 426 509 B1

Figure 5b

Figure 6a

Figure 6b

Figure 7a

Figure 7b

Figure 7c

*Figure 8a*

*Figure 8b*

Figure 8c

EP 2 426 509 B1

Figure 9

Figure 10

EP 2 426 509 B1

*Figure 11*

*Figure 12*

Figure 13

EP 2 426 509 B1

Figure 14

Figure 15

Figure 16a

Figure 16b

Figure 16c

Figure 17

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020050169 A